# EUROPEAN PATENT SPECIFICATION

(11) **EP 1 997 500 B1**
(45) Date of publication and mention of the grant of the patent: **02.01.2013**
(21) Application number: 08157135.8
(22) Date of filing: 29.05.2008
(51) Int. Cl.: A61K 36/16, A61K 36/79, A61K 36/074, A61K 36/73, A61K 36/48, A61K 36/8962, A61K 36/344, A61K 36/42, A61K 36/8969, A61K 36/44, A61P 25/32

(54) **Health tea and method for preparing the same**
Gesundheitstee und Verfahren für seine Zubereitung
Thé bon pour la santé et son procédé de préparation

(30) Priority: 29.05.2007 KR 20070052145; 30.04.2008 KR 20080040230
(43) Date of publication of application: 03.12.2008
(73) Proprietor: Jang, Young-Sam, Mapo-Ku Seoul 121-210 (KR)
(72) Inventor: Jang, Young-Sam, Mapo-Ku Seoul 121-210 (KR)
(74) Representative: ABG Patentes, S.L.

(56) References cited:
- WO-A-00/71145
- US-A1- 2006 045 929
- DATABASE WPI Week 200516 Thomson Scientific, London, GB; AN 2005-143423 XP002493834 & CN 1 541 705 A (FU M) 3 November 2004 (2004-11-03)
- DATABASE WPI Week 200243 Thomson Scientific, London, GB; AN 2002-395442 XP002493835 & CN 1 338 289 A (PAN N) 6 March 2002 (2002-03-06)
- DATABASE WPI Week 199727 Thomson Scientific, London, GB; AN 1997-289831 XP002493836 & CN 1 103 313 A (XU X) 7 June 1995 (1995-06-07)
- DATABASE WPI Week 200448 Thomson Scientific, London, GB; AN 2004-500437 XP002493837 & CN 1 491 686 A (HE C) 28 April 2004 (2004-04-28)
- DATABASE WPI Week 200531 Thomson Scientific, London, GB; AN 2005-296983 XP002493838 & CN 1 562 264 A (CAI X) 12 January 2005 (2005-01-12)
- DATABASE WPI Week 200561 Thomson Scientific, London, GB; AN 2005-592221 XP002493839 & CN 1 596 683 A (XIAO S) 23 March 2005 (2005-03-23)
- DATABASE WPI Week 200319 Thomson Scientific, London, GB; AN 2003-195566 XP002493840 & KR 2002 081 995 A (DRIGO CO LTD) 30 October 2002 (2002-10-30)

## Description

### BACKGROUND OF THE INVENTION

### 1. Field of the Invention

The present invention relates to natural health and wellness teas and preparation methods thereof, more particularly, to a health tea prepared using a variety of natural materials, which is beneficial to the human body and has advantageous effects of the materials preferably contained in the health tea, as well as a method for preparation of the same.

### 2. Description of the Related Art

In recent years, inorganic or organic harmful materials and/or toxic chemicals produced or exhausted from factories during industrial manufacturing processes cause environmental pollution and threaten natural environments and the survival of various animal species including human beings, and in turn, are gradually destroying the harmony of natural ecosystems.

Especially, there is a significant problem that waste water leakage generated by landfill of domestic waste causes heavy metal and/or hazardous material-based contamination of groundwater around the landfill. If a person drinks such contaminated groundwater, toxic ingredients usually accumulate in the body and cause various adult diseases and, ultimately, result in fatal damage to the body.

Although modern people mostly take lots of fatty foods, especially, meat, and suffer from adult diseases such as hypertension, diabetes, obesity, etc., there is still proposed no potent drinks with specific efficacy on such adult diseases.

The human body comprises about 70% of water and water is necessary for proper body operation and/or physical activity. Therefore, research studies on drinking water and other beverages such as various teas are currently conducted in association with environmental pollution.

According to recent development trends of drinking products, modern people show great interest in different types of drinking products rather than traditionally known simple favorite beverages, thereby reflecting much concern for health.

There are many studies that improve or enhance performance of drinking products through pharmacological effects of herbal drug plants, and such drinking products are preferably prepared by adding herbal drug ingredients to any specific favorite beverages. As a result, some of the products with specific pharmacological efficacy have developed and are now commercially available in the market.

Due to bad drinking habits (sometimes called Korean drinking culture), many troubles and shameful conducts often occur regardless of time and place. According to recent statistical data, it is indicated that drink-driving kills about 1,100 people on roads and injures 5,500 each year and, in addition to, may cause enormous loss for the national economy.

Immoderate drinking causes mental and/or physical damage to drinkers and is even being considered as a form of indirect murder thus being considered a serious crime, because it may injure others as well as the drinkers themselves.

Accordingly, in order to considerably reduce drinking derived damages, there is still a requirement of developing improved natural health teas that effectively overcome health problems such as hangover or fatigue and enhance blood circulation.

As herbal teas containing Korean medical plants as primary ingredients, ginseng tea, Lycium Chinese fruit tea, Acanthopanax sessiliflorus root bark extract tea, Cassia tora seed extract tea and so on are traditionally known and other types of teas are being newly and continuously developed.

However, the above traditional herbal teas normally comprise single ingredient based herbal plants as principal ingredients. The herbal teas containing specific herbal plants are partially used for drinking purposes of consumers since the herbal plants have originally known pharmacological properties and are expected to exhibit these pharmacological properties in the teas. But, the herbal teas lack widespread appeal due to inferior taste or flavor compared to coffee, black tea and/or green tea. Also, there are few consumers convinced that the herbal plants contained in the teas really exhibit original pharmacological properties thereof.

Since the herbal teas also generally have a strong bitter taste, a lack of preference and require high price, there is a difficulty in habitually drinking or having the herbal teas, which are different from commercially available drinking products or teas.

### SUMMARY OF THE INVENTION

Accordingly, the present invention is directed to solve problems of conventional arts as described above and, an object of the present invention is to provide natural health teas that are prepared using a variety of natural materials (such as herbal plants), which have advantages including: improvement of health such as hangover removal, recovery of fatigue, enhanced blood circulation and the like; economical benefits in manufacturing the health teas by using natural plants; and preferable extraction of desired ingredients having medical effects by carrying the materials in separate bags and heating the bags, respectively, as well as a method for preparation of the health teas.

In order to accomplish the above object, the present invention provides a health tea comprising: 4 to 6% by weight (hereinafter, abbrev. to "wt.%") of ginkgo leaves; 2 to 4wt.% of Codonopsis lanceolata; 1 to 2wt.% of Ganoderma lucidum; 3 to 5wt.% of Solomon's seal; 1 to 3wt.% of roots of sprout beans; 4 to 6wt.% of cucumber; 1 to 2wt.% of onion; 2 to 4wt.% of Hedysarum; 1 to 3wt.% of Maximowiczia typica; 4 to 6wt.% of pear fruits; 2 to 4wt.% of red beans; 6 to 8wt.% of persimmon leaves; 6 to 8wt.% of persimmon fruits; 1 to 3wt.% of sugar; and the balance of water relative to total 100wt.% of the health tea, which is prepared by heating to extract desired ingredients of the raw materials.

The present invention further provides a method for preparation of a natural health tea, comprising the steps of: placing at least one bag containing ginkgo leaves, Codonopsis lanceolata, Ganoderma lucidum, Solomon's seal, roots of sprout beans, cucumber, onion, Hedysarum, Maximowiczia typica, pear fruits, red beans, persimmon leaves and persimmon fruits in water; heating the mixture at 100 to 150°C for 24 to 36 hours to extract desired herbal ingredients; and adding sugar to the mixture containing the extracted material.

Preferably, ginkgo leaves, Codonopsis lanceolata and Ganoderma lucidum are placed in the first bag while Solomon's seal, roots of sprout beans, cucumber and onion are fed into the second bag. Similarly, Hedysarum, Maximowiczia typica and pear fruits are added to the third bag and the fourth bag contains red beans, persimmon leaves and persimmon fruits. All of the contents in the bags are treated in water by the heating extraction step.

As described above, the health tea of the present invention is obtained using a variety of natural herbal plants so as to exhibit beneficial effects before and/or after drinking, such as hangover removal, recovery of fatigue, enhanced blood circulation, etc.

Because of using natural herbal plants, the inventive health tea can be economically manufactured at relatively low price.

Also, since each of the natural herbal plants contained in the bags are heated, the present inventive method can preferably extract herbal ingredients with desired medical effects from the materials.

### BRIEF DESCRIPTION OF THE DRAWINGS

These and other objects, features, aspects, and advantages of the present invention will be more fully described in the following detailed description of preferred embodiments and examples, taken in conjunction with the accompanying drawings. In the drawings:
Fig. 1 is a cross sectional view showing a preparation device using a method for preparation of a health tea according to an embodiment of the present invention;
Fig. 2 is a flow diagram showing the method for preparation of a health tea according to an embodiment of the present invention.

### DETAILED DESCRIPTION OF THE INVENTION

Hereinafter, preferred embodiments of the present invention will be described in more detail by the following examples with reference to the accompanying drawings.

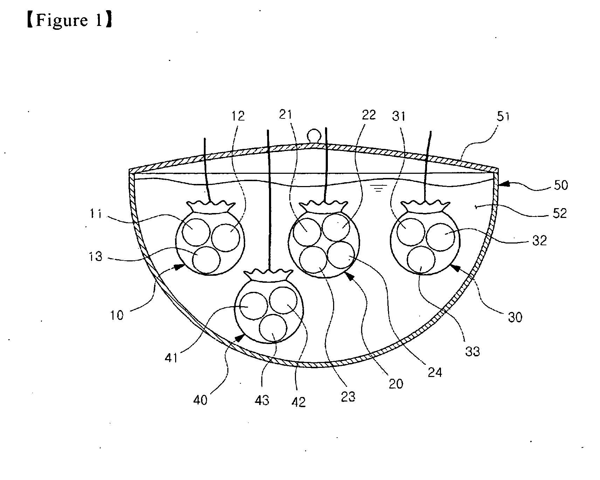

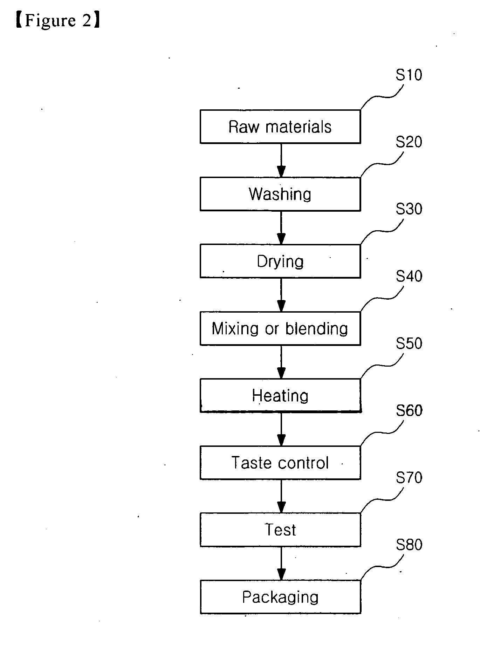

Fig. 1 is a cross sectional view showing a preparation device using a method for preparation of a health tea according to an embodiment of the present invention, while Fig. 2 is a flow diagram illustrating the above preparation method.

As illustrated in Fig. 1 and Fig. 2, the health tea according to the embodiment of the present invention comprises natural herbal plants, preferably, ginkgo leaves, Codonopsis lanceolata, Ganoderma lucidum, Solomon's seal, roots of sprout beans, cucumber, onion, Hedysarum, Maximowiczia typica, pear fruits, red beans, persimmon leaves, persimmon fruits, sugar and water, which is obtained by heating for extraction of desired herbal ingredients.

With regard to the health tea of the present invention, fresh and green ginkgo leaves mostly have excellent medical effects, for example, improved blood circulation to treat arteriosclerosis and heart diseases, treatment of digestive organ diseases such as dysentery, stomach pain, diarrhea, and so on. More particularly, ginkgo leaf derived extracts are well known to enhance the heart and/or alleviate thrombosis so as to preferably function as a medical tea.

The method of the present invention prepares ginkgo leaf materials by cleanly washing fresh and green ginkgo leaves, drying the leaves in the shade and slicing up the dried leaves.

An amount of the leaf materials preferably ranges from 4 to 6wt.% since less than 4wt.% of the materials shows little efficacy in improving blood circulation while more than 6wt.% may cause toxic poisoning from contaminants by excessive intake of the leaves.

Codonopsis lanceolata homogenously includes protein, fat, hydrocarbons and others and, especially, hydrocarbons with large contents of potassium, calcium and vitamin B groups. Therefore, Codonopsis lanceolata can make the body warm and supplement vitality, show beneficial effects to the liver, strengthen the stomach and bowels to improve digestive function so that Codonopsis lanceolata is used as one of medicated food products, and assist function of the lungs and kidneys.

The inventive method prepares Codonopsis lanceolata materials by grubbing up natural Codonopsis lanceolata plants during a specific period from autumn to early spring before sprouting, trimming off fine roots and smoothing stems of the plants, washing the smoothed materials, drying the washed materials in sunlight, and slicing the dried materials to have an average size of 5cm.

An amount of the Codonopsis lanceolata materials preferably ranges from 2 to 4wt.% since less than 2wt.% of the materials shows little efficacy of nutrition supplementary food products while more than 4wt.% has stronger bitter taste.

Ganoderma lucidum is an annual mushroom generated every year from roots of broad-leaved trees during the summer season and sometimes sprouted on ground, which has gloss such as lacquer or varnish on a surface of hat and stem of the mushroom. Ganoderma lucidum contains in general ergosterol, citric acid, polysaccharide, mannitol, methamine, amino acid, benzoic acid and the like.

Ganoderma lucidum can promote vitality of human body, strengthen bones and muscles and activate blood circulation to allow a person to stay awake or to have a fresh and clear mind. This plant also has medical effects to improve condition of the liver, or to treat some diseases including, for example: adult diseases such as hypertension, diabetes or obesity; joint disease; nervous breakdown; insomnia; dyspnea; ophthalmopathy, etc. More preferably, Ganoderma lucidum has superior efficacy to remedy hemorrhoids and gastrointestinal diseases.

The inventive method prepares Ganoderma lucidum materials by cleanly washing natural Ganoderma lucidum mushrooms and slicing up the mushrooms to have an average diameter ranging from 2 to 3cm.

An amount of the Ganoderma lucidum materials preferably ranges from 1 to 2wt.% since less than 1wt.% of the materials shows little efficacy while more than 2wt.% may cause adverse effects when an excessive dose of the materials is applied to a person.

Solomon's seal is well known to clean blood and make five main organs such as liver, kidneys, heart, spleen and lungs in comfort, and long term oral use of this plant results in that the user feels refreshed and light in his(her) body and has improved and healthy complexion. This plant also exhibits excellent effect to restore sexual vigor in old age. Solomon's seal includes nutrients and active components to inhibit ageing and has beneficial effects for physically weak persons after dieting, or persons who suffer from emotional hunger who always feel hunger.

The inventive method prepares Solomon's seal materials by grubbing up roots and stems of natural Solomon's seal plants during early spring or late autumn, cleanly washing the plants, drying the washed plants in the shade, slicing up the dried plants, and gently roasting the sliced materials in a cauldron until the materials become yellow.

An amount of the Solomon's seal materials preferably ranges from 3 to 5wt.% since less than 3wt.% of the materials shows little efficacy while more than 5wt.% may be heavy on the stomach and/or even weaken the body.

Roots of sprout beans containing plenty of asparagine are well known to promote generation of enzymes for alcohol degradation in the liver, thereby demonstrating excellent efficacy in hangover removal.

An amount of the prepared materials preferably ranges from 1 to 3wt.% since less than 1wt.% of the materials shows little efficacy while more than 3wt.% may adversely affect the body of a person who has weak kidney function.

Cucumber is well known to effectively reduce edema and remove a hangover and/or alcoholic poisoning and also has medical effects in reducing symptoms caused by cardiac edema, renal edema or metabolic disorders. Especially, cucumber tea is effective for an upset stomach or vomiting after drinking excessive alcohol and/or severe headache.

The inventive method prepares cucumber materials by washing and peeling the cucumber, cutting the cucumber into pieces and completely drying the pieces in the shade.

An amount of the prepared cucumber materials preferably ranges from 4 to 6wt.% since less than 4wt.% of the materials shows little efficacy while more than 6wt.% may be heavy on the stomach of a person who has weak and cold stomach and bowels.

Onion is a root of a biennial Liliaceae plant and contains calcium, iron, vitamin B2 and C, carotene, citric acid, malic acid, etc. so as to assist blood circulation similar to garlic and prevents clotting of blood to inhibit thrombosis. This plant also has beneficial effects in improvement of digestive functions and treatment of hypertension and/or arteriosclerosis.

The inventive method prepares onion materials by peeling and washing natural onions, cutting the onions into pieces and drying the pieces in the shade.

An amount of the onion materials preferably ranges from 1 to 2wt.% since less than 1wt.% of the materials shows less control effect of blood circulation while more than 2wt.% has a very strong smell.

Hedysarum has excellent beneficial effects to supplement vitality such as fatigue recovery or to support stamina or energy of a person suffering from hyperhidrosis (that is, excessive sweating).

The inventive method prepares Hedysarum materials by grubbing up natural Hedysarum plants during the autumn season, removing heads and fine roots of the plants and drying the plants in sunlight.

An amount of the Hedysarum materials preferably ranges from 2 to 4wt.% since less than 2wt.% of the materials shows less efficacy while more than 4wt.% may adversely affect the body of a person who has weak kidney function.

Maximowiczia typica is known to have five tastes including sweet taste, sour taste, bitter taste, salty taste and hot taste and, especially, stronger sour taste. This plant comprises various components such as Schizandrin, Gomisin, citral, malic acid, citric acid, etc. and is effective to strengthen the heart, decrease blood pressure and increase immune activity, thereby being effectively used as nutrients and/or tonics. Alternatively, this plant protects bronchus, reinforces pulmonary function, and has antitussive effect and expectorant effect to treat cough, sputum and/or thirst.

The inventive method prepares Maximowiczia typica materials by drying fruits of natural Maximowiczia typica trees.

An amount of the Maximowiczia typica materials preferably ranges from 1 to 3wt.% since less than 1wt.% of the materials shows little efficacy in protecting the bronchus while more than 3wt.% may cause excessive sweating and adversely affect the body of a person.

Pear is a fruit of pear tree effective for protection of the bronchus and remedy of bronchial diseases so as to preferably treat colds, cough, asthma and the like. The pear fruit also has medical effects to promote bowel movement and diuretic action or renal function. Moreover, the pear fruit reduces sputum and cough, effectively alleviates symptoms when a person gets hoarse, suffers from stomach ache and/or the stomach is cold, and assists medical treatment of abscesses. Beyond these, this fruit exhibits detoxification to remove a hangover.

An amount of the pear fruits preferably ranges from 4 to 6wt.% since less than 4wt.% of the fruits shows less efficacy in protecting the bronchus while more than 6wt.% may cause excessive sweating and adversely affect the body of a person.

Red beans are well known to remove waste materials from the body and an edema and have superior efficacy in hangover removal.

An amount of the red beans preferably ranges from 2 to 4wt.% since less than 2wt.% of the red beans shows less efficacy in removing a hangover while more than 4wt.% may be heavy on the stomach of a person who has a weak and cold stomach and bowels.

Persimmon leaves include vitamin C in great quantities and are effectively used to remove a hangover and, especially, vitamin C contained in persimmon leaf tea is difficult to destroy so as to be preferably absorbed into the body. Moreover, these leaves contain calcium, tannic acid ingredients, etc. so that the leaves can promote diuretic action or renal function and, in addition, reduce blood pressure and arteriosclerosis while reinforcing immune activity.

The inventive method prepares persimmon leaf materials by preferably obtaining natural fresh leaves of persimmon trees in about May to June every year, cleanly washing and drying the leaves in the shade for 2 to 3 days, and finely slicing the dried leaves. Alternatively, after steaming the completely dried leaves, the steamed materials are again air-dried in the shade with air circulation and stored in a dry place away from moisture.

If the persimmon leaves are infused to drink after obtaining and completely drying, 'tannin' ingredients in the leaves protect the stomach by contraction of gastric mucosa and show efficacy in hangover removal.

An amount of the persimmon leaf materials preferably ranges from 6 to 8wt.% since less than 6wt.% of the materials shows less efficacy while more than 8wt.% may increase acidity and be heavy on the stomach of a person who has a weak stomach and bowels.

Persimmon fruits are well known to have efficacy in hangover removal. Especially, since the persimmon fruits principally comprise fructose and vitamin C in great quantities which are useful for promoting oxidation and decomposition of alcohol absorbed in the body, these show superior efficacy in protecting and removing a hangover.

In addition, tannic acid contained in the persimmon fruits exhibits medical effects including, for example: treatment of gastrointestinal diseases such as gastric ulcers or diarrhea; treatment of cerebral hemorrhage by hemostatic activity of tannic acid; and capillary reinforcement leading to excellent protection and treatment of circulatory diseases such as hypertension.

An amount of the persimmon fruits preferably ranges from 6 to 8wt.% since less than 6wt.% of the fruits shows less efficacy while more than 8wt.% may be heavy on the stomach of a person who has a weak stomach and bowels.

Sugar is added to control sweet taste of the health tea according to the present invention. An amount of sugar preferably ranges from 1 to 3wt.% since less than 1wt.% of sugar shows less efficacy in controlling the sweet taste while more than 3wt.% may cause adult diseases and/or dental diseases.

Water is essential to form a human body that comprises about 70% of water. A constant amount of water should be fed to the body every day for proper body operation. More particularly, it is presumed that living of the human body is a result of complicated chemical reactions generated by a water solution comprising a variety of ingredients.

The water used to produce the health tea according to the present invention preferably includes clean water such as first grade pure water, ground water, etc.

As illustrated in Fig. 1, the health tea according to an embodiment of the present invention was obtained by preparing desired materials, selectively mixing or blending together the materials, and placing the mixtures in the first bag 10, the second bag 20, the third bag 30 and the fourth bag 40, respectively.

Each of the bags was immersed in a cauldron 50 containing water 52. After putting a lid 51 on the cauldron to seal it, the cauldron 50 was subjected to heating extraction at 100 to 150°C for 24 to 36 hours while applying pressure thereto. After heating all of the materials placed in the cauldron, constant amounts of sugar was added to the heated product to control taste of the health tea.

More particularly, the first bag 10 included ginkgo leaves 11, Codonopsis lanceolata 12 and Ganoderma lucidum 13 while the second bag 20 included Solomon's seal 21, roots of sprout beans 22, cucumber 23 and onion 24. Also, the third bag 30 included Hedysarum 31, Maximowiczia typica 32 and pear fruits 33 while the fourth bag 40 included red beans 41, persimmon leaves 42 and persimmon fruits 43. The selective mixing or blending of the raw materials as described above is preferable to prevent modification or alteration of beneficial effects of the raw materials during heating extraction.

The bag is preferably made of natural fabrics such as hemp cloth that allows each of the materials to be sufficiently infused so as to extract desired ingredients, allows water 52 to easily flow in or out through the bag while preventing loss of the materials, and inhibits modification of the ingredients.

Heating temperature of the cauldron 50 preferably ranges from 100 to 150°C since less than 100°C of the heating temperature is not enough to infuse the materials and obtain the desired ingredients while more than 150°C may cause modification or degeneration of effects of the materials.

Heating time of the cauldron 50 preferably ranges from 24 to 36 hours since less than 24 hours is not enough to infuse the materials and obtain the desired ingredients while more than 36 hours may cause degeneration of the materials or deterioration of taste or flavor.

Referring to Fig. 2, the method for preparation of the health tea according to an example of the present invention will be described in more detail below.

First of all, raw materials were prepared (step S10), which included: 5wt.% of ginkgo leaves; 3wt.% of Codonopsis lanceolata; 1wt.% of Ganoderma lucidum; 4wt.% of Solomon's seal; 2wt.% of roots of sprout beans; 5wt.% of cucumber; 1wt.% of onion; 3wt.% of Hedysarum; 2wt.% of Maximowiczia typica; 5wt.% of pear fruits; 3wt.% of red beans; 7wt.% of persimmon leaves; and 7wt.% of persimmon fruits.

After washing all of the materials (step S20), each of the materials was preferably dried in the shade or in sunlight (step 30).

The dried materials were selectively mixed or blended together and fed into the bags, respectively (step S40). Each of the bags containing the mixtures was placed in the cauldron, followed by addition of water in relation to the total weight of the materials, then, heating extraction of desired ingredients from the materials at 100 to 150°C for 24 to 36 hours after putting a lid on the cauldron (step S50).

2wt.% of sugar was added to the heat extracted solution in order to control taste of the health tea (step S60). The resulting health tea was subjected to examination of ingredients (step S70).

The health tea passing the examination is typically poured into disposable sterile packages, vacuum packaged by means of an aseptic packaging machine and shipped or distributed to customers (step S80).

### EXAMPLE 1: Health tea product

With regard to the health tea of the present invention prepared by the above described method, experiments for ingredients and quality standards thereof were carried out and the results are shown as follows:
(1) color : light yellow
(2) taste : a little sweet and smooth going down the throat of a user
(3) tar dyes : not detected
(4) heavy metal : less than 3ppm
(5) preservation period : more than 2 years of safety guarantee for products

### EXAMPLE 2: Clinical experiments for health tea product

With regard to the health tea of the present invention prepared by the above described method, clinical experiments for medical effects of the tea were carried out by having or taking the tea before and after drinking liquor and the results are shown as follows:
(1) Having tea after drinking liquor:
150 adult men with constant age distributions between ages 25 and 45 years as shown in Table 1 drank liquor as usual, for example, 2 or 3 bottles of liquor per each of them. After 10 minutes, everyone nearly lost consciousness, felt dizzy such that they could not keep their balance, experienced dulled thinking and was about to vomit. To each of them, about 140 Mℓ of the inventive health tea was given to determine beneficial effects of the tea. The results are shown in Table 2 below.

**TABLE 1**

| Age distribution | Ages 25 to 30 years | Ages 31 to 35 years | Ages 36 to 40 years | Ages 41 to 45 years | Sum |
|---|---|---|---|---|---|
| Number of persons | 35 | 35 | 45 | 35 | 150 |

**TABLE 2**

| Drinking level | Number of persons | Excellent efficacy of tea | Good efficacy of tea | Less efficacy of tea | Rate of efficacy (%) |
|---|---|---|---|---|---|
| Blind drunk | 120 | 118 | 2 | 0 | 100% |
| Slightly drunk | 30 | 30 | 0 | 0 | 100% |
| Sum | 150 | 148 | 2 | 0 | |

The cases with excellent efficacy of the tea were determined in that vomiting was first stopped 10 minutes after having the health tea, then, they gradually recovered consciousness to become sober.
The cases with good efficacy of the tea were determined in that they gradually recovered consciousness and did not feel dizzy after having the tea.
The cases with little efficacy of the tea were determined in that drinking symptoms had not disappeared even after having the tea.
(2) Having tea before drinking liquor:
Each of 150 adult men with constant age distributions between ages 25 and 45 years as shown in Table 1 had about 140 Mℓ of the inventive health tea 10 minutes before drinking liquor as usual. They underwent experiments for beneficial effects of the health tea. The results are shown in Table 3 below.

**TABLE 3**

| Drinking condition | Able to drink more than usual | Mildly drunk | No effect | Sum | Rate of efficacy (%) |
|---|---|---|---|---|---|
| Number of persons | 115 | 35 | 0 | 150 | 100% |

As understood from the above results, it was demonstrated that since most of participants in these experiments were mildly drunk or could drink more than usual, the present inventive health tea was preferably effective to remove a hangover after drinking.

Accordingly, it is clearly understood that the health tea of the present invention preferably comprising roots of sprout beans, cucumber, red beans, persimmon leaves and fruits has beneficial effects in removing a hangover after drinking liquor. The health tea further includes ginkgo leaves, Ganoderma lucidum and onion so as to improve blood circulation before and after drinking. Moreover, the health tea additionally contains Codonopsis lanceolata, Solomon's seal, Hedysarum, pear fruits and Maximowiczia typica, thereby effectively restoring or recovering fatigue before and after drinking.

## Claims

1. A method for preparation of a health tea, comprising the steps of:
a) placing at least one bag containing 4 to 6wt.% of ginkgo leaves, 2 to 4wt% of Codonopsis lanceolata, 1 to 2wt.% of Ganoderma lucidum, 3 to 5wt.% of Solomon's seal, 1 to 3wt.% of roots of sprout beans, 4 to 6wt.% of cucumber, 1 to 2wt.% of onion, 2 to 4wt.% of Hedysarum. 1 to 3wt.% of Maximowiczia typica, 4 to 6wt.% of pear fruits, 2 to 4wt.% of red beans, 6 to 8wt.% of persimmon leaves, 6 to 8wt.% of persimmon fruits in water and the balance of water relative to total 100wt.% of the raw materials;
b) heating the mixture at 100 to 150°C for 24 to 36 hours to extract desired herbal ingredients;
c) and adding 1 to 3wt.% sugar to the mixture containing the extracted material.

2. The method according to claim 1, wherein ginkgo leaves, Codonopsis lanceolata and Ganoderma lucidum are placed in the first bag; Solomon's seal, roots of sprout beans, cucumber and onion are fed into the second bag; Hedysarum, Maximowiczia typica and pear fruits are placed in the third bag: and red beans, persimmon leaves and persimmon fruits are contained in the fourth bag, all of these materials in the first to fourth bags being treated in water during the heating extraction step.

3. A health tea prepared by a method as defined in any of claims 1 or 2.

4. A dry herbal composition comprising ginkgo leaves, Codonopsis lanceolata. Ganoderma lucidum, Solomon's seal, roots of sprout beans, cucumber, onion, Hedysarum, Maximowiczia typica, pear fruits. red beans, persimmon leaves and persimmon fruits.

## Patentansprüche

1. Verfahren zur Herstellung eines Gesundheitstees, das folgende Schritte umfasst:
a) Einbringen mindestens eines Beutels, der 4 bis 6 Gewichtsprozentanteile Ginkgoblätter, 2 bis 4 Gewichtsprozentanteile Codonopsis lanceolata, 1 bis 2 Gewichtsprozentanteile Ganoderma lucidum, 3 bis 5 Gewichtsprozentanteile Salomonssiegel, 1 bis 3 Gewichtsprozentanteile Sprossenbohnenwurzeln, 4 bis 6 Gewichtsprozentanteile Gurke, 1 bis 2 Gewichtsprozentanteile Zwiebel, 2 bis 4 Gewichtsprozentanteile Hedysarum, 1 bis 3 Gewichtsprozentanteile Maximowiczia typica, 4 bis 6 Gewichtsprozentanteile Birnenfrüchte, 2 bis 4 Gewichtsprozentanteile Rote Bohnen, 6 bis 8 Gewichtsprozentanteile Kakiblätter sowie 6 bis 8 Gewichtsprozentanteile Kakifrüchte enthält, in Wasser, mit einem Restanteil Wasser bezogen auf 100 Gewichtsprozentanteile der Rohmaterialien;
b) Erwärmen der Mischung bei 100 bis 150 °C für 24 bis 36 Stunden, um die gewünschten pflanzlichen Bestandteile zu extrahieren;
c) und Hinzufügen von 1 bis 3 Gewichtsprozentanteilen Zucker zu der Mischung, die das extrahierte Material enthält.

2. Verfahren nach Anspruch 1, wobei Ginkgoblätter, Codonopsis lanceolata und Ganoderma lucidum in den ersten Beutel gegeben werden; Salomonssiegel, Sprossenbohnenwurzeln, Gurke und Zwiebel in den zweiten Beutel gefüllt werden; Hedysarum, Maximowiczia typica und Birnenfrüchte in den dritten Beutel gegeben werden; und Rote Bohnen, Kakiblätter und Kakifrüchte in dem vierten Beutel enthalten sind, wobei all diese Materialien in den ersten bis vierten Beuteln während des Schrittes der Extrahierung durch Erwärmen in Wasser behandelt werden.

3. Ein Gesundheitstee, hergestellt durch ein Verfahren wie in einem der Ansprüche 1 oder 2 definiert.

4. Eine trockene pflanzliche Zusammensetzung, die Ginkgoblätter, Codonopsis lanceolata, Ganoderma lucidum, Salomonssiegel, Sprossenbohnenwurzeln, Gurke, Zwiebel, Hedysarum, Maximowiczia typica, Birnenfrüchte, Rote Bohnen, Kakiblätter und Kakifrüchte enthält.

## Revendications

1. Méthode de préparation d'un thé médicinal, comprenant les étapes de :
a) placer au moins un sachet contenant 4 à 6% en poids de feuilles de gingko, 2 à 4% en poids de *Codonopsis lanceolata,* 1 à 2% en poids de *Ganoderma lucidum,* 3 à 5% en poids de sceau de Salomon, 1 à 3% en poids de racines de graines germées, 4 à 6% en poids de concombre, 1 à 2% en poids d'oignons, 2 à 4% en poids d'Hédysarum, 1 à 3% en poids de *Maximowiczia typica,* 4 à 6% en poids de poires, 2 à 4% en poids de haricots rouges, 6 à 8% en poids de feuilles de plaqueminier, 6 à 8% en poids de fruits de plaqueminier dans de l'eau et équilibrer en eau par rapport à un total de 100% en poids de matières brutes :
b) chauffer le mélange entre 100 et 150°C pendant 24 à 36 heures pour extraire les ingrédients désirés,
c) et ajouter 1 à 3% en poids de sucre au mélange contenant le matériau extrait.

2. Méthode selon la revendication 1, dans laquelle les feuilles de gingko, le *Codonopsis lanceolata* et le *Ganoderma lucidum* sont placés dans le premier sachet, le sceau de Salomon, les racines de graines germées, le concombre et l'oignon sont placés dans le deuxième sachet, l'Hédysarum, le *Maximowiczia typica* et les poires sont placés dans le troisième sachet, et les haricots rouges, les feuilles de plaqueminier et les fruits de plaqueminier sont contenus dans le quatrième sachet, tous ces matériaux dans les sachets 1 à 4 étant traités dans de l'eau pendant l'étape d'extraction par chauffage.

3. Thé médicinal préparé selon une méthode telle que définie dans l'une quelconque des revendications 1 ou 2.

4. Composition sèche à base de plantes comprenant des feuilles de gingko, du *Codonopsis lanceolata,* du *Ganoderma lucidum,* du sceau de Salomon, des racines de graines germées, du concombre, de l'oignon, de l'Hédysarum, du *Maximowiczia typica,* des poires, des haricots rouges, des feuilles de plaqueminier et des fruits de plaqueminier.
